# EUROPÄISCHE PATENTSCHRIFT

(11) **EP 2 649 968 B1**
(45) Veröffentlichungstag und Bekanntmachung des Hinweises auf die Patenterteilung: **06.04.2016**
(21) Anmeldenummer: 13003500.9
(22) Anmeldetag: 27.01.2009
(51) Int. Cl.: A61F 2/68, A61F 2/66

(54) **Verfahren zur Steuerung eines orthopädischen Fußteils und das orthopädische Fußteil**
Method for controlling an orthopaedic foot component and orthopaedic foot component
Procédé de commande d'une elément de pied orthopédique et l'elément de pied orthopédique

(30) Priorität: 07.02.2008 DE 102008008282
(43) Veröffentlichungstag der Anmeldung: 16.10.2013
(62) Teilanmeldung aus: 09001070.3
(73) Patentinhaber: Otto Bock HealthCare GmbH, 37115 Duderstadt (DE)
(72) Erfinder: Kaltenborn, Sven, DE - 37115 Duderstadt (DE); Ruess, Felix, 2201 Gerasdorf (AT); Zarling, Sven, DE - 37115 Duderstadt (DE); Bischof, Bernhard, AT - 1160 Wien (AT); Kraus, Thomas, AT - 2263 Dürnkrut (AT)
(74) Vertreter: Lins, Edgar

(56) Entgegenhaltungen:
- WO-A-03/086245
- WO-A-2006/000211
- WO-A-2008/048658
- WO-A-2008/103917
- US-A1- 2005 197 717
- US-A1- 2007 043 449

## Beschreibung

Die Erfindung betrifft ein orthopädisches Fußteil mit einem Unterschenkel-Anschlussteil, einem als Knöchelgelenk wirkenden Drehgelenk, mit dem ein Fußteil des Fußes in Richtung Dorsalflexion und Plantarflexion drehbar mit dem Anschlussstück verbunden ist, mit einer die Drehbewegung um das Drehgelenk beeinflussenden Dämpfungsanordnung, mit einer Sensoranordnung zur Erkennung von Aktionszuständen des künstlichen Fußes und mit einer mit der Sensoranordnung verbundenen Steuerung, die die Dämpfungsanordnung steuert. Die Erfindung betrifft ferner ein Verfahren zur Steuerung eines derartigen orthopädischen Fußteils.

Es ist bekannt, dass die Steuerung eines orthopädischen Fußteils, in Form eines künstliches Fußes oder einer Fußorthese, zahlreiche Anforderungen erfüllen sollte, um eine sichere Benutzung des Fußes und einen möglichst natürlichen Bewegungsablauf beim Gehen des Prothesenträgers zu ermöglichen. So ist es beispielsweise wünschenswert, während des Gehens beim Fersenauftritt ein gesteuertes Aufsetzen des Fußteils auf die Standebene und ein kontrolliertes Überrollen Ober den Vorfuß zu ermöglichen, wobei der Körper des Prothesenträgers etwas angehoben wird. In der Schwungphase führt der natürliche Fuß eine Dorsalflexion aus, wodurch eine Längenverkürzung des Beines - und damit einer erleichtertes Durchschwingen - erreicht wird. Ferner ist für ein sicheres Stehen mit einem künstlichen Fuß erforderlich, dass eine hohe Stabilität des Winkels zwischen dem Fußteil und dem Unterschenkel-Anschlussteil besteht. Eine hierfür sinnvolle Blockierung des als Knöchelgelenk wirkenden Drehgelenks des künstlichen Fußes sollte jedoch einstellbar sein, damit das Stehen auf einem schrägen Untergrund oder auf einem Schuh mit einer größeren Absatzhöhe für den Prothesenträger in entspannter Weise möglich ist.

Es ist beispielsweise durch US 2005/0197717 A1 bekannt, den Knöchelwinkel während eines Gangzyklus durch einen Aktuator in Form eines Doppelschraubenmotors zu steuern. Verwendet wird dabei ein Fußteil, das durch eine definierte Elastizität zur Realisierung eines annähernd natürlichen Gangbildes beitragen soll. Der Einsatz eines Aktuätors in Form eines ständig angetriebenen Motors benötigt erhebliche Mengen elektrischer Energie, die dem künstlichen Gelenk zugeführt werden muss. Hierfür muss der Prothesenträger eine leistungsfähige Batterie mit sich tragen, die aufgrund der benötigten hohen Kapazität großvolumig und schwer sein muss.

Es ist ferner bekannt, für die Erfüllung von Teilaspekten der Steuerung ausschließlich eine Dämpfung der Drehbewegung des Fußteils relativ zum Anschlussteil zu steuern. Durch US 7,029,500 B2 ist hierfür ein spezielles Drehgelenk als Knöchelgelenk des künstlichen Fußes vorgesehen, wobei die Dämpfung mit einer magneto-rheologischen Flüssigkeit beeinflusst wird, indem die Ausübung eines Magnetfeldes die Flüssigkeit von einer niedrigen Viskosität auf eine hohe Viskosität - und damit die Dämpfung von einer niedrigen Dämpfung auf eine starke Dämpfung - umschaltbar ist. Dadurch gelingt es, eine beim Gehen sich automatisch einstellende Dorsal- bzw. Plantarflexion zu verriegeln und für eine bestimmte Zeit beizubehalten. Ferner wird erreicht, dass im Sitzen das Gelenk freigegeben wird, um die Einstellung einer Plantarflexion, wie sie auch der natürliche Fuß beim Sitzen ausführt, einzustellen.

Durch US 2002/0138153 A1 ist es ferner bekannt, zur Einstellung des Ruhewinkels im Stehen bei einem schrägen Untergrund oder für unterschiedliche Absatzhöhen eines Schuhs, der mit dem künstlichen Fuß verwendet wird, eine Hydraulikanordnung zu verwenden, bei der die Flüssigkeit bei einer Plantarflexion von einem Flüssigkeitsvorrat in den anderen und für eine Dorsalflexion in entgegen gesetzter Richtung strömt. In eine Verbindungsleitung zwischen den beiden Flüssigkeitsreservoirs ist eine elektromagnetische Spule eingesetzt, die die Viskosität der als Hydraulikflüssigkeit verwendeten magnetorheologischen Flüssigkeit steuert, wodurch eine Umschaltung der Dämpfung von einem ersten Dämpfungspegel auf einen zweiten Dämpfungspegel möglich ist. Um die bestellten Steuerungsaufgaben zu erfüllen, wird dabei ein Neigungssensor für das Unterschenkelteil gegenüber der Lotrechten und ein Bodenkontaktsensor verwendet. Der Bodenkontaktsensor zeigt an, dass der künstliche Fuß durch Auftreten auf den Boden eine Bodenreaktionskaft erfährt.

US 2007/043449 A1 offenbart eine aktive Steuerung, bei der somit das Hauptfußteil im Knöchelgelenk mit einem Aktuator gedreht werden kann, um beispielsweise eine motorgestützte Plantarflexion durchzuführen. Darüber hinaus können Dämpfungen, beispielsweise für die Dorsiflexion eingestellt werden. Für das Fußteil sind Sensorsysteme offenbart, mit denen die Steuerung in Abhängigkeit von Arbeitszuständen des Fußteils erfolgen. Erwähnt ist ein Trägheitsnavigationssystem mit Beschleunigungsmessern und Gyroskopen sowie Positiönssensoren, Kraftsensoren, Geschwindigkeitssensoren und Auslenkungssensoren, mit denen Bewegungen oder Position des Knöchelgelenks von Energie speichernden Federn, Geschwindigkeiten von Dämpfern usw. gemessen werden.

WO 2006/000211 A2 offenbart einen künstlichen Fuß, der an ein Unterschenkel-Anschlussteil anschließbar ist und ein Hauptfußteil aufweist, das um ein Knöchelgelenk drehbar ist, wobei an das vordere Ende des Hauptfußteils ein Vorderfußteil drehbar angeschlossen Ist, dessen Winkelstellung relativ zum Hauptfußteil sich in Abhängigkeit von der Winkelstellung des Unterschenkelteils einstellt. Sensoren für die Erfassung von Funktionszuständen sind nicht erwähnt.

Der vorliegenden Erfindung liegt die Aufgabe zugrunde, ein orthopädisches Fußteil der eingangs erwähnten Art so steuerbar auszugestalten, dass mehr. Anforderungen an die Eigenschaften eines orthopädischen Fußteils als bisher mit einer ausschließlich passiven Steuerung der Dämpfung der Bewegung um das Knöchelgelenk erfüllbar sind.

Diese Aufgabe wird erfindungsgemäß mit einem Verfahren zur Steuerung eines orthopädisches Fußteils, das ein Unterschenkel-Anschlussteil, ein als Knöchelgelenk wirkendes Drehgelenk, mit dem ein Fußteil in Richtung Dorsalflexion und Plantarflexion drehbar mit dem Anschlussteil verbunden ist, eine die Drehbewegung um das Drehgelenk beeinflussende Dämpfungsanordnung, eine Sensoranordnung zur Erkennung von Aktionszuständen des orthopädischen Fußteils und eine mit der Sensoranordnung verbundene Steuerung aufweist, die die Dämpfungsanordnung steuert, dadurch gelöst, dass ein zum am Knöchelgelenk auftretenden Drehmoment proportionaler Wert, der Knöchelwinkel zwischen Anschlussteil und Fußteil und der Absolutwinkel des Fußteils bezogen auf die Lotrechte bestimmt werden, dass in Abhängigkeit von diesen gemessenen Werten das Abrollen des Fußes in der Standphase des Gehens, die Stellung des Fußteils in der Schwungphase des Gehens und die Stellung und Bewegbarkeit des Fußteils während des Stehens ausschließlich mittels der Dämpfungsanordnung gesteuert wird, dass die Beeinflussung von Parametern eines Gangzyklus durch für diesen Gangzyklus bereits gemessene Sensorsignale erfolgt und dass ein Nulldurchgang des Knöchelmoments als Kriterium bei den Messungen verwendet wird. Wenn sich an eine Plantarflexion innerhalb eines vorgegebenen Zeitintervalls keine Dorsalflexion anschließt, wird zum Stehen gewechselt.

Dabei wird vorzugsweise die Dämpfung in Richtung Dorsalflexion und die Dämpfung in Richtung Plantarflexion mit separaten Dämpfungsmitteln separat gesteuert. Die Dämpfungsmittel können dabei vorzugsweise während des Gehvorgangs in zahlreiche Zwischenstellungen, vorzugsweise kontinuierlich, verstellt werden. Die Verstellung erfolgt dabei durch ein Programm der Steuerung zur vollständigen Bewegungssteuerung, die vorzugsweise nicht durch Materialelastizitäten bewirkt wird. Wesentlich ist dabei, dass die Verstellung der Dämpfungsmittel unter Berücksichtigung des gemessenen Knöchelwinkels oder Absolutwinkels erfolgen kann.

Die erfindungsgemäße Steuerung der Bewegung des Fußes erlaubt die Beeinflussung von Parametern eines Gangzyklus durch für diesen Gangzyklus bereits gemessene Sensorsignale. Die Adaption kann daher ummittelbar erfolgen, sodass die Parameter eines Gangzyklus nicht - wie bei bisherigen aktiven Steuerungen bekannt - durch gemessene Parameter eines vorhergehenden Gangzyklus gesteuert werden müssen. So ist es beispielsweise erfindungsgemäß ohne weiteres möglich, unter Berücksichtigung des Absolutwinkels des Fußteils (bzw. unter Berücksichtigung des Signals des Drehwinkelsensors auch des Unterschenkel-Anschlussteils) und des Messsignals des Drehmomentsensors eine Schrittlängenbestimmung beim Aufsetzen der Ferse auf den Untergrund durchzuführen und in Abhängigkeit von der bestimmten Schrittlänge die Dämpfungsparameter für den gerade eingeleiteten Gangzyklus zu bestimmen. In Abhängigkeit von der bestimmten Schrittlänge können beispielsweise die von einem bestimmten Nullpunkt in Richtung Dorsalflexion und Plantarflexion zulässigen Drehwinkel des Knöchelgelenks variiert werden. Die Begrenzung der Drehbewegung des Knöchelgelenks erfolgt durch ein entsprechendes Zusteuern des jeweiligen Dämpfungsmittels in Abhängigkeit von dem gemessenen Knöchelgelenkwinkel.

Die erfindungsgemäße Kombination von Messsignalen erlaubt auch das Unterscheiden zwischen Gehen und Stehen durch eine geeignete Auswertung dieser Messsignale durch einen Prozessor in der Steuerung. Der Übergang vom Gehen in das Stehen wird nämlich dann festgestellt, wenn das Knöchelmoment von einem negativen Wert ausgehend einen Wert nahe Null erreicht und der Absolutwinkel Null beträgt und beibehalten wird. Beim entspannten Stehen steht der Unterschenkel bzw. die Schwerpunktslinie des Körpers in der Lotrechten. Der Übergang vom Stehen zum Gehen wird dadurch festgestellt, dass das Knöchelmoment nahe Null ist, der Absolutwinkel eine vom Stehen abweichende Schwelle überschreitet und die Änderung des Absolutwertes über die Zeit eine Schwelle überschreitet. Dabei kann die Messung des Absolutwinkels, die das Vorschwingen des Beines beim Beginn des Gehens wiedergibt, bereits zur indirekten Bestimmung der Schrittlänge verwendet werden, da der beim Vorschwingen des Unterschenkels auftretende Absolutwinkel zur Schrittlänge proportional ist.

Das erfindungsgemäße Verfahren ermöglicht ferner eine geeignete Steuerung des künstlichen Fußes während des Gangzyklus, was unten noch näher erläutert werden wird.

Es stellt sich heraus, dass die Verwendung der nur drei Sensorenarten eine Steuerung des künstlichen Fußes sowohl während eines Gangzyklus als auch während des Stehens in einer gewünschten Weise nur durch Einwirkung auf die Dämpfung der Bewegung des Knöchelgelenks ermöglicht. Wie unten näher erläutert wird, reichen die nur wenigen Sensorsignale aus, um die benötigten unterschiedlichen Aktionszustände des Fußes eindeutig zu ermitteln und den Fuß so einzustellen, dass er die Bewegung eines natürlichen, gesunden Fußes während des Gangzyklus imitieren und trotzdem während des Stehens, auch auf geneigten Untergründen oder mit unterschiedlichen Absatzhöhen, ein sicheres Standgefühl vermitteln kann. Es zeigt sich ferner, dass die Signale der erfindungsgemäß vorgesehenen Sensoren ferner ausreichen, alltägliche Gangsituationen, wie das Herauf- und Herabgehen von Treppen und Rampen und das Rückwärtsgehen ebenfalls zuverlässig gesteuert werden können.

In einer bevorzugten Ausführungsform weist die Dämpfungsanordnung separate und separat steuerbare Dämpfungsmittel in Richtung Plantarflexion einerseits und Dorsalflexion andererseits auf. Demgemäß sind die Bewegungen in beiden Richtungen separat steuerbar. Dies ermöglicht eine unterschiedliche Behandlung der Dämpfung in den,unterschiedlichen Drehrichtungen und beispielsweise in Abhängigkeit von dem Ausgangssignal des Knöchelwinkelsensors.

Bei dem erfindungsgemäßen orthopädischen Fußteil ist das Fußteil vollständig oder zumindest überwiegend starr ausgebildet. Die federnde Bewegung des Fußteils in der Standphase wird durch die Steuerung der Dämpfung der Drehbewegung des Fußteils relativ zum Unterschenkel-Anschlussteil realisiert. Hierfür ist es erforderlich, dass die Dämpfungsanordnung in mehrere Zwischenzustände einstellbar ist, vorzugsweise kontinuierlich verstellbar ist. Die Verstellung der Dämpfung wird durch ein entsprechendes Programm in der Steuerung gesteuert, sodass die Bewegung des Fußteils relativ zum Unterschenkel-Anschlussteil vollständig kontrolliert erfolgt. Die Verwendung von elastischen Teilen, die stoßartige Übergänge vermeiden, beispielsweise beim Fersenauftritt durch ein elastisches Fersenteil, ist dadurch nicht ausgeschlossen.

Eine Variation ergibt sich daraus, dass das Fußteil in einer besonders bevorzugten Ausführungsform der Erfindung aus einem über das Knöchelgelenk mit dem Anschlussteil verbundenen starren Basisteil und einem mit dem Basisteil gelenkig verbundenen Vorderfußteil besteht. Das drehgelenkig am vorderen Ende des Basisteils angelenkte Vorderfußteil imitiert die Bewegung der Zehen eines natürlichen Fußes in den Zehengelenken, wobei der von den beiden Ventilen gesteuerte Dämpfungszylinder einerseits am Unterschenkel-Anschlussteil und andererseits am Vorderfußteil in der Nähe des Drehgelenks zwischen Basisteil und Vorderfußteil angelenkt wird. Je nach Winkelstellung des Vorderfußteils kommt es dabei zu einer mehr oder weniger stark untersetzten Übertragung der Dämpfungswirkung des Dämpfungszylinders auf die Drehbewegung des Basisteils relativ zu dem Unterschenkel-Anschlussteil. Anders ausgedrückt verändert sich die für das Knöchelgelenk wirksame Hebellänge des Basisteils durch das Abknicken des Vorderfußteils, beispielsweise beim Überrollen in der Standphase des Gangzyklus. Diese definierte Verkürzung der Hebellänge des Basisteils wird vom Programm in der Steuerung berücksichtigt.

Die separate Steuerung der Dämpfungsmittel in Richtung Plantarflexion einerseits und Dorsalflexion andererseits ermöglicht die Festlegung einer Nullpunktstellung, von der aus die Bewegung in den beiden Richtungen gesteuert wird. Diese Nullpunktstellung kann manuell durch einen Schalter am Knöchelgelenk festgelegt werden. Der Schalter kann ein Fernbedienungsschalter sein, sodass die Betätigung nicht am Fuß erfolgen muss.

Eine derartige Einstellung des Nullpunktes ist beispielsweise sinnvoll, wenn der Träger des orthopädischen Fußteils dieses mit anderen Schuhen mit einer geänderten Absatzhöhe verwendet. Auf diese Weise kann durch die manuelle Einstellung des Nullpunktes eine Anpassung an die neue Absatzhöhe unproblematisch vorgenommen werden. Alternativ ist es aber auch möglich, dass die Steuerung die Nullpunktstellung aus den Daten der Sensoren erkennt und dass die Dämpfungsmittel von der Nullpunktstellung ausgehend steuerbar sind.

In einer in der Praxis bevorzugten Ausführungsform ist die Dämpfungsanordnung eine in einer Hydraulikflüssigkeit durchströmbare Hydraulikanordnung, bei der vorzugsweise zur Beeinflussung der Strömung in Richtung Dorsalflexion ein Dorsalventil und in Richtung Plantarflexion ein Plantarventil vorgesehen ist. Das orthopädische Fußteil weist in dieser bevorzugten Ausführungsform somit zwei Einwegventile auf, durch die die Hydraulikflüssigkeit jeweils nur in einer Richtung strömen kann, um so die separate Steuerbarkeit der Bewegungen in beiden Richtungen zu ermöglichen.

Die beschriebene Ausbildung des künstlichen Fußes mit einem Fußteil, das aus einem Basisteil und einem gelenkig mit dem Basisteil verbundenen Vorderfußteil besteht, ermöglicht die ansonsten nicht mögliche Unterscheidung zwischen einer Absatzhöhe eines Schuhs und einem entsprechend geneigten Untergrund.

Während das Vorderfußteil bei einem geneigten Untergrund in einer durch eine Rückstellfeder vorgespannten gestreckten Lage verbleibt, führt ein Schuh mit einem hohen Absatz zu einer abgewinkelten Stellung des Vorderfußteils gegenüber dem Basisteil. Ein zusätzlicher Winkelsensor, der die Winkelstellung des Vorderfußteils relativ zum Basisteil wiedergibt, könnte daher die Unterscheidung zwischen "geneigtem Untergrund" und "Absatzhöhe" bewirken.

Ist ein derartiger Sensor nicht vorgesehen, kann der Tatsache, dass die Einstellung auf die Absatzhöhe lediglich beim Anziehen des Schuhs zu erfolgen hat, dadurch Rechnung getragen werden, dass zu diesem Zeitpunkt ein Signal auf die Steuerung übertragen wird, mit dem die Nullpunktstellung des Drehgelenks für den momentanen Stehzustand, der durch die Absatzhöhe verursacht wird, festgestellt wird. Demzufolge ist für die Steuerung ein Schaltsignal vorgesehen, das vorzugsweise mit einer Fernbedienungseinrichtung von dem Prothesenträger auf die Steuerung übertragbar ist.

Vorzugsweise wird als Dämpfungsanordnung eine Hydraulikanordnung verwendet, die von einer Hydraulikflüssigkeit durchflossen wird, wobei die Dämpfungsmittel, die für die beiden Bewegungsrichtungen separat vorgesehen und gesteuert werden, durch hydraulische Einwegventile (Plantarventil und Dorsalventil) gebildet werden.

Die erfindungsgemäß verwendete Sensoranordnung erlaubt im Übrigen die Feststellung des jeweiligen Nullpunktes, sodass eine Adaption an den neuen Nullpunkt bereits während des Gangzyklus selbst vorgenommen werden kann.

Die Erfindung soll im Folgenden anhand eines in der Zeichnung dargestellten Ausführungsbeispiels näher erläutert werden. Es zeigen:
- Figur 1: eine schematische Darstellung eines ersten Ausführungsbeispiels einer erfindungsgemäßen Fußprothese;
- Figur 2: eine schematische Darstellung eines zweiten Ausführungsbeispiels einer erfindungsgemäßen Fußprothese;
- Figur 3: eine schematische Darstellung eines dritten Ausführungsbeispiels einer erfindungsgemäßen Fußprothese;
- Figur 4: eine Draufsicht auf eine konstruktiv detaillierte weitere Ausführungsform einer erfindungsgemäßen Fußprothese;
- Figur 5: einen Vertikalschnitt parallel zur Sagittalebene durch die Fußprothese gemäß Figur 4.

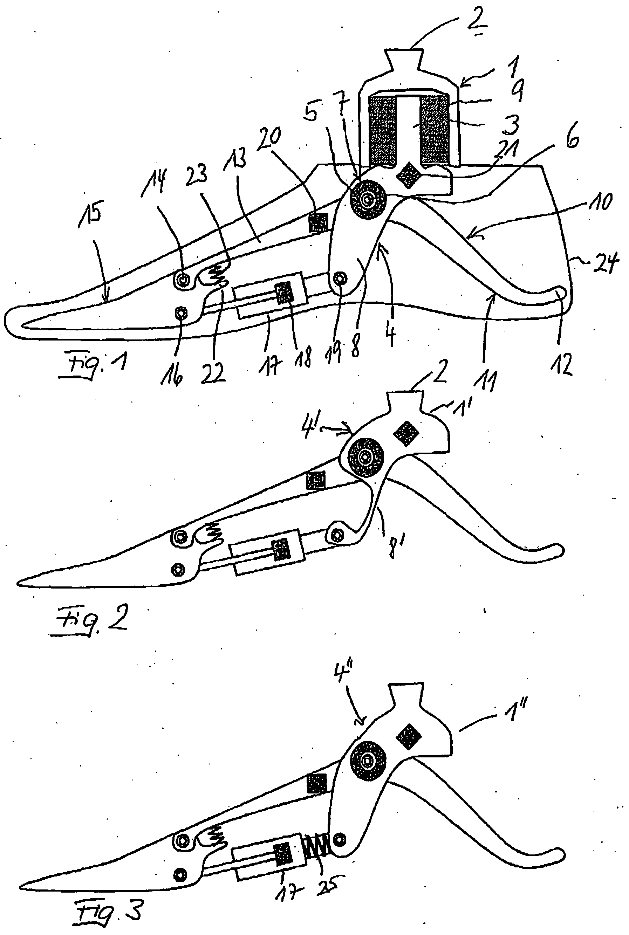

Bei dem in Figur 1 dargestellten Ausführungsbeispiel ist ein Befestigungsstück 1 mit einem Justieransatz 2 In Form eines umgekehrten Pyramidenstumpfes mit vier Schrägflächen ausgebildet. Das Befestigungsstück 1 bildet einen nach unten offenen Topf, in den ein nach oben ragender Steg 3 eines zweiarmigen Hebels 4 hineinragt. Der zweiarmige Hebel ist um ein Drehgelenk 5 drehbar, dessen Drehachse 6 zugleich die Achse eines Knöchelgelenks des künstlichen Fußes bildet. Das Drehgelenk 5 ist mit einem Winkelsensor 7 versehen. Der zweiarmige Hebel 4 weist einen starren, nach unten ragenden Ansatz 8 auf.

In dem nach unten offenen Topf des Befestigungsstücks 1 ist der mit dem Steg 3 gebildete Zwischenraum durch ein relativ steifes, elastisches Material 9 ausgefüllt, sodass die Bewegung des Befestigungsstücks 1 nur etwas bedämpft auf Bewegung des Stegs 3 des zweiarmigen Hebels 4 übertragen wird. Demgemäß vollzieht der Ansatz 8 die Bewegung des Befestigungsstücks 1 nach, jedoch aufgrund des elastischen Materials 9 etwas bedämpft,

Das das Knöchelgelenk bildende Drehgelenk 5 trägt ferner ein Hauptfußteil 10, das sich ebenfalls ein zweiarmiger Hebel mit einem hinteren Hebelarm 11 in den Fersenbereich des Fußes hinein erstreckt, wo der schräg nach hinten und unten verlaufende hintere Hebelarm 11 mit einem etwa horizontal abgerundeten Ende 12 versehen ist.

Das Hauptfußteil weist einen vom Knöchelgelenk 5 nach vorn erstreckten vorderen Hebelarm 13 auf, der sich nahezu geradlinig vom Knöchelgelenk 5 nach vorn etwas schräg nach unten verlaufend erstreckt, sodass das Hauptfußteil 10 zum Knöchelgelenk 5 hin nach oben gewölbt ausgebildet ist und vom Knöchelgelenk 5 nach hinten in den Fersenbereich sowie nach vorne in einen Vorderfußbereich hinein schräg abfällt, wobei der schräge Abfall in den Fersenbereich hinein steiler ist als in den Vorderfußbereich.

Der vordere Hebelarm 13 des Hauptfußteils 10 endet am Beginn des Vorderfußbereichs und trägt dort ein Drehgelenk 14, mit dem ein einen Zehenbereich nachbildendes Vorderfußteil 15 drehbar an dem vorderen Hebelarm 13 des Hauptfußteils angelenkt ist. Das Drehgelenk 14 weist eine Drehachse auf, die parallel zur Drehachse 6 des Knöchelgelenks 5 in horizontaler Richtung verläuft. Da das Vorderfußteil 15 den Zehenbereich eines natürlichen Fußes imitiert, ist es nach vorne hin dreieckförmig mit einer Spitze auslaufend ausgebildet. Unterhalb des Drehgelenks 14 befindet sich am Vorderfußteil 15 ein weiteres Drehgelenk 16, mit dem eine Kolbenstange eines Kolbens 18 eines Hydraulikzylinders 17 an dem Vorderfußteil 15 angelenkt ist. Der Hydraulikzylinder 17 ist mit einem Drehgelenk 19 drehbar an dem freien Ende des nach unten ragenden Ansatzes 8 des zweiarmigen Hebels 4 angelenkt, sodass sich das Drehgelenk 19 unterhalb und etwas nach vorn (in Richtung Vorderfußbereich 15) versetzt zum Knöchelgelenk 5 angeordnet ist.

Das Knöchelgelenk 5 enthält den Winkelsensor 7 für die Messung des Knöchelwinkels, also des Winkels zwischen dem (fluchtend mit dem Unterschenkel ausgerichteten) Steg 3 und dem vorderen Hebelarm 13 des Hauptfußteils 10.

Der vordere Hebelarm 13 des Hauptfußteils 10 trägt ferner einen Neigungssensor 20, der die Neigung relativ zur Erdanziehungskraft (relativ zur Lotrechten) ermittelt. Derartige Neigungssensoren 20, die einen absoluten Neigungswinkel relativ zur Erdbeschleunigung bestimmen, sind als Beschleunigungssensoranordnungen mit oder ohne Gyroskop bekannt.

Der zweiarmige Hebel 4 enthält fluchtend mit dem Justieransatz 2, d.h. fluchtend mit dem (künstlichen) Unterschenkel des Patienten einen Knöchelmomentsensor 21, der das an dieser Stelle wirkende Drehmoment misst.

Das Vorderfußteil 15 ist an seinem hinteren Ende mit einem Lageransatz 22 versehen, der zum Halten einer auf Zug und Druck belastbaren Feder 23 dient, die sich mit ihrem anderen Ende am vorderen Hebelarm 13 des Hauptfußteils 10 abstützt. Die Feder 23 bewirkt eine Rückstellung des Vorderfußteils 15 nach einer Dorsalflexion, wobei die Rückstellgeschwindigkeit durch den Hydraulikzylinder 17 bestimmt wird.

Der Hydraulikzylinder 17 kann als passiver Aktuator ausgebildet sein, in dem der vom Kolben 18 bewirkte Hydraulikfluss durch (nicht dargestellte) Ventile gesteuert wird, wobei die Ventile selbst nicht nur ein- und ausschaltbar sind, sondern auch für eine definierte Durchflussmenge gesteuert werden können. Es ist aber auch möglich, den Hydraulikzylinder 17 als aktiven Aktuator auszubilden, der ohne Krafteinwirkung von außen eine Verstellung des Vorderfußteils 15 bewirken kann.

Die in Figur 2 dargestellte Ausführungsform entspricht im Wesentlichen der Ausführungsform gemäß Figur 1. Ein Unterschied besteht darin, dass das Befestigungsstück 1' mit dem Justieransatz 2 einstückig ausgebildet ist, sodass eine durch das elastische Material 9 gebildete Elastizität nicht mehr vorhanden ist. Stattdessen ist der nach unten ragende Ansatz 8' des zweiarmigen Hebels 4' mit einer Materialverdünnung ausgebildet, sodass das das Drehgelenk 19 tragende freie Ende des Ansatzes elastisch federnd zum restlichen Material des zwelarmigen Hebels 4' angeordnet ist.

Selbstverständlich weist der künstliche Fuß gemäß der zweiten Ausführungsform ebenso einen kosmetischen Überzug 24 auf, wie die erste Ausführungsform. Dieser kosmetische Überzug 24 ist für die zweite und dritte Ausführungsform jedoch nicht wiederholt dargestellt.

Bei dem künstlichen Fuß gemäß der in Figur 3 dargestellten dritten Ausführungsform ist der zweiarmige Hebel 4" ebenfalls einstückig mit dem Justieransatz 2 ausgebildet. Auch der nach unten ragende Ansatz 8 des zweiarmigen Hebels 4" ist starr wie bei der ersten Ausführungsform. Stattdessen ist der Hydraulikzylinder 17 über eine Spiralfeder 25 elastisch mit dem nach unten ragenden Ansatz 8 des zweiarmigen Hebels 4" verbunden. Dadurch wird in Serie mit der Wirkung des Hydraulikzylinders 17 eine Elastizität verwirklicht, die bei der in Figur 1 dargestellten Ausführungsform durch das elastische Material 9 und bei der in Figur 2 dargestellten Ausführungsform durch den federnden Ansatz 8' realisiert ist. Alle übrigen Teile der dritten Ausführungsform entsprechen denen der ersten Ausführungsform.

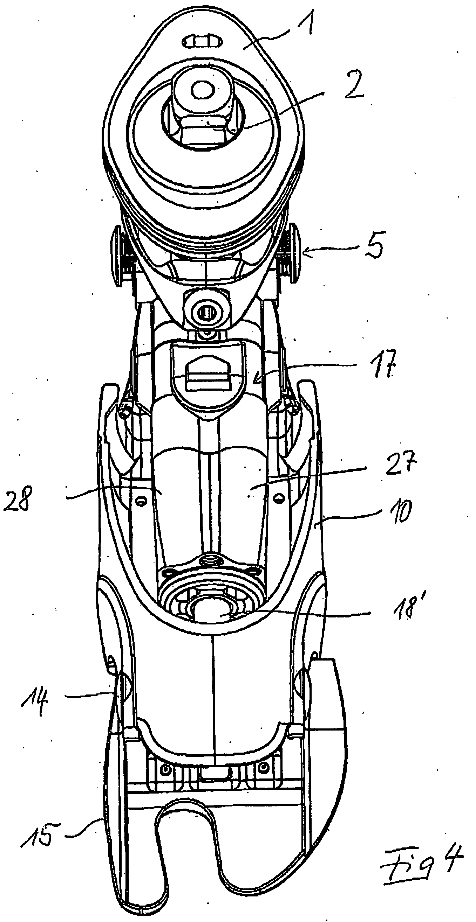

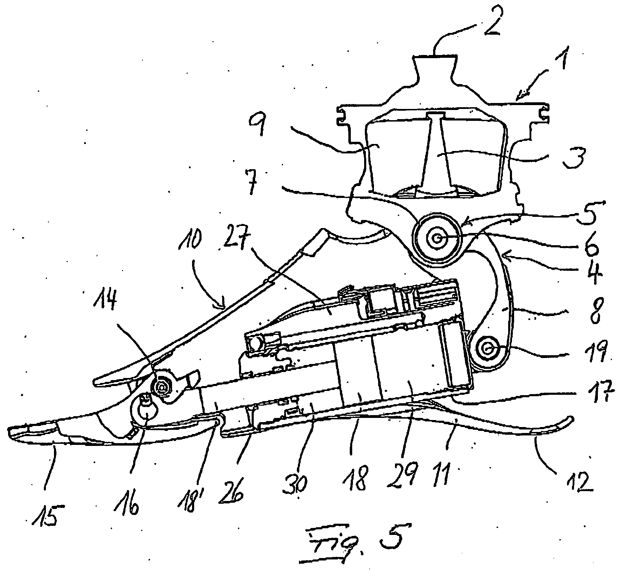

Das in den Figuren 4 und 5 dargestellte Ausführungsbeispiel lässt das Befestigungsstück 1 mit dem pyramidenförmigen Justieransatz 2 erkennen. In dem Befestigungsstück 1 befindet sich das elastische Material 9, das mit dem nach oben ragenden Steg 3 des zweiarmigen Hebels 4 dämpfend zusammenwirkt. Der nach unten ragende Ansatz 8 des zweiarmigen Hebels 4 verläuft in diesem Ausführungsbeispiel in Gehrichtung hinter dem Knöchelgelenk 5 und Ist dort über das Drehgelenk 19 an dem Hydraulikzylinder 17 angelenkt. In dem Hydraulikzylinder 17 bewegt sich längsverschiebbar der Kolben 18, der über ein im Hydraulikzylinder 17 befindliches Lager 26 herausgeführt und mit dem weiteren Drehgelenk 16 des Vorderfußteils 15 verbunden ist. Das Knöchelgelenk 5 lagert ferner das Hauptfußteil 10, das hier in Form eines starren Gehäuses ausgebildet ist und einen nach hinten gerichteten elastischen Hebel 11 als Fersenhebel aufweist. Das Fußteil 10 ist mit dem Fersenhebel 11 somit gemeinsam um das Knöchelgelenk 5 relativ zu dem Befestigungsstück 1 und zu dem zweiarmigen Hebel 4 schwenkbar. Die Schwenkbewegung zwischen dem Befestigungsstück 1 und dem Fußteil 10 wird über den zweiarmigen Hebel 4 und den Hydraulikzylinder 17 gesteuert und bedämpft. Die Anlenkung der Kolbenstange 18' des Kolbens 18 an dem Vorderfußteil 15 bewirkt dabei lediglich eine zusätzliche Steuerung des die Zehenplatte bildenden Vorderfußteils 15, die jedoch die Steuerung des Hauptfußteils 10 nur geringfügig modifiziert, da das weitere Drehgelenk 16 in unmittelbarer Nähe des Drehgelenks 14 zwischen dem Vorderfußteil 15 und dem Hauptfußteil 10 angeordnet ist. Der Hydraulikzylinder 17 ist mit zwei Steuerventilen 27, 28 versehen, die auf der Oberseite des Hydraulikzylinders 17 angeordnet sind. Die Steuerventile 27, 28 sind mit Kammern 29, 30 des Hydraulikzylinders 17 beiderseits des Kolbens 18 verbunden, wobei (nicht dargestellte) Rückschlagventile dafür sorgen, dass durch das erste Steuerventil 27 lediglich ein Fluss der Hydraulikflüssigkeit von der unteren Kammer 29 zur vorderen Kammer 30 erfolgen kann, wodurch die Einschubbewegung des Kolbens 18 in den Hydraulikzylinder 17 ermöglicht wird, was einer Plantarflexion des Hauptfußteils 10 gegenüber dem Befestigungsstück 1 entspricht. Der andere Steuerzylinder 28 ermöglicht mit Rückschlagventilen lediglich den Hydraulikfluss von der vorderen Kammer 30 in die hintere Kammer 29, wodurch ein Herausziehen des Kolbens 18 aus dem Hydraulikzylinder 17 ermöglicht wird, also eine Verlängerung des Abstandes zwischen den Drehgelenken 19, 16 stattfindet. Dies entspricht einer Dorsalflexion zwischen dem Befestigungsstück 1 und dem Hauptfußteil 10. Gleichzeitig wird durch die Verschiebung des Drehgelenks 16 gegenüber dem Drehgelenk 14 nach vorn ein Anheben des Vorderfußteils 15 bewirkt.

Die Funktion des künstlichen Fußes ist für die dargestellten Ausführungsformen gleich. Mit der Sensoranordnung zur Messung des Knöchelwinkels, des Knöchelmoments und des absoluten Neigungswinkels lassen sich die relevanten Funktionszustände des künstlichen Fußes ermitteln und differenzieren, wobei das Signal des Knöchelwinkelsensors zur Ermittlung des Knöchelwinkels (zwischen Befestigungsstück 1, 1', 1" und Hauptfußteil 10) einerseits und die jeweilige Knöchelwinkelgeschwindigkeit andererseits ausgewertet werden.

So kann beispielsweise die Erkennung, ob mit dem künstlichen Fuß gegangen wird oder gestanden wird, dadurch erfolgen, dass die Knöchelwinkelgeschwindigkeit im Nulldurchgang des Knöchelmoments bestimmt wird. Liegt die Knöchelwinkelgeschwindigkeit im Nulldurchgang des Knöchelmoments unter einem Schwellwert, wird dies als "Stehen" erkannt und der Aktuator in Form des Hydraulikzylinders 17 mittels der Ventile auf einen hohen Widerstand eingestellt, durch den ein Dorsalanschlag gebildet werden kann.

Die Erkennung einer abschüssigen Neigung oder der Absatzhöhe wird durch den Neigungssensor 20 im Mittelfußbereich des Hauptfußteils 10 beim Nulldurchgang des Knöchelmoments bestimmt.

Wird das Gehen in der Ebene detektiert, wird das Ventil, das für die Plantarflexion des Fußes zuständig ist, in einer halboffenen Stellung belassen, während das Ventil, das die Dorsalfelxion bestimmt, mit zunehmenden Knöchelwinkel geschlossen wird, um einen Dorsalanschlag zu bilden.

Wird ein Gehen bergauf detektiert, wird dabei eine erweiterte Dorsalflexion des Vorderfußteils 15 zugelassen.

Wird beim Gehen der Fersenauftritt nach der Schwungphase und zu Beginn der Standphase insbesondere durch ein negatives Knöchelmoment detektiert, wird das Ventil für die Plantarflexion so gesteuert, dass es mit zunehmenden Knöchelwinkel in Richtung der Plantarflexion schließt und somit einen Anschlag für die Plantarflexion bildet.

Wird ein Zehenabstoß am Ende der Standphase detektiert (abfallendes Knöchelmoment bei vergrößertem Knöchelwinkel), wird das Ventil für die Dorsalflexion nach einer Totzeit vollständig geöffnet, um durch ein elastisches Element die Anhebung des Vorderfußteils (Zehenanhebung) in der Schwungphase auszulösen.

An diesen Beispielen ist erkennbar, dass die wichtigen Steuerungen eines künstlichen Fußes beim Stehen und beim Gehen auch in Abhängigkeit von der Bodenneigung oder der Absatzhöhe zutreffend vorgenommen werden können, wobei die Steuerung des Bewegungswiderstandes über den Hydraulikzylinder bereits ausreicht. Alternativ kann aber auch ein aktiver Aktuator im Sinne eines Stellgliedes vorgesehen werden.

In einem Ausführungsbeispiel für die Erkennung der Bewegungszustände für die Fußprothese und die daraus resultierende Steuerung werden folgende Funktionen realisiert:

### Unterscheidung Stehen-Gehen

Die Unterscheidung zwischen Stehen und Gehen erfolgt mit folgenden Kriterien:
1. Erkennung einer Schwungphase
   Eine Schwungphase wird dadurch erkannt, dass das Knöchelmoment nahe Null ist, da der Fuß in der Schwungphase unbelastet ist.
   Der Absolutwinkel des Fußteils 10 überschreitet einen Schwellenwert, der für das Stehen individuell definiert sein kann. Ferner überschreitet die Absolutwinkelgeschwindigkeit einen definierten Schwellenwert.
2. Erkennen eines Fersenauftritts im vorgeschwungenen Zustand
   Es wird ein negatives Knöchelmoment (plantarflektierend) detektiert. Das Absolutwinkelsignal entspricht einem vorgeschwungenen Fuß im Vergleich zu einem für das Stehen individuell definierten Schwellenwert.
   Optional kann eine Plantarflexion beim Fersenauftritt über die Knöchelwinkelgeschwindigkelt angezeigt werden.
3. Rückkehr zum Stehen
   Nach einem detektierten Fersenauftritt verbleibt der Absolutwinkel des Fußteils 10 innerhalb eines für das Stehen individuell definierten Schwellenwertes. Alternativ oder ergänzend kann eine aktive Umkehr der Bewegungsrichtung in der mittleren Standphase von dorsal nach plantar als Kriterium für das Stehen erkannt werden.
   Beim detektierten Stehen werden die Steuerventile 27, 28 so eingestellt, dass sich für das Stehen Anschläge nach ventral und dorsal für einen engen Winkel (Nullpunktlage) ergeben. Für den Gangzyklus wird der Anschlag nach dorsal verschoben und die Dämpfungseigenschaften für die Plantar- und Dorsalflexion in Abhängigkeit von der Schrittlänge eingestellt.

### Unterscheidung Ebene-Rampe

Der gemessene Absolutwinkel zu Beginn der mittleren Standphase im Gehzyklus, also nach dem Aufsetzen des vollen Fußes auf den Untergrund, ist größer oder kleiner als ein für das Gehen in der Ebene definierter Wertebereich für den Absolutwinkel.

Entsprechend der festgestellten Neigung der Rampe wird der Dorsalanschlag verändert und die Dämpfungseigenschaften bei der Plantarflexion und der Dorsalflexion werden in Abhängigkeit des Absolutwinkels und der prognostizierten Schrittlänge eingestellt.

### Erkennung Rückwärtsgehen

Die Erkennung des Rückwärtsgehens besteht aus einer Erkennung der Rückschwungphase und der Erkennung eines Vorderfußauftritts in dem rückgeführten Zustand.
1. Erkennung einer Rückschwungphase
   Bei einem gemessenen Knöchelmoment nahe Null entspricht das Absolutwinkelsignal einem gegenüber dem Stehen zurückgeführten Fuß (Retroversion) und die Absolutwinkelgeschwindigkeit überschreitet einen definierten Schwellenwert.
2. Erkennung des Vorderfußauftritts im rückgeführten Zustand Gemessen wird ein größeres positives Knöchelmoment.
   In Abhängigkeit von den gemessenen Werten wird der Anschlag nach dorsal verstellt und die Dämpfungseigenschaften bei Plantar- und Dorsalflexion werden in Abhängigkeit vom Absolutwinkel bei dem Vorfußauftritt eingestellt.

### Anpassung an unterschiedliche Absatzhöhen

Vorzugsweise wird die Absatzhöhe durch das Auslesen des Absolutwinkelsignals während eines manuell ausgelösten Triggersignals festgestellt. Proportional zum Absolutwinkel wird der Nullpunkt für die Steuerventile 27, 28 eingestellt.

Alternativ hierzu ist es möglich, die Absatzhöhe von einer Rampenneigung bei einem künstlichen Fuß mit einem gelenkig angeschlossenen Vorderfußteil 15 dadurch zu bestimmen, dass der Winkel des Vorderfußteils 15 in Relation zum Hauptfußteil 10 gemessen wird. Hierin liegt eine zusätzliche Option im Rahmen der vorliegenden Erfindung.

### Stehen auf geneigtem Untergrund

Gemessen wird der Absolutwinkel bei einer Umkehr der Bewegungsrichtung von plantar nach dorsal, wenn das Knöchelmoment einen Nulldurchgang erfährt. Dementsprechend wird der dorsale Anschlag für die Steuerung des Hydraulikzylinders 17 mit den Steuerventilen 27, 28 in Abhängigkeit von der Untergrundneigung verstellt.

### Erkennung Treppengehen

Wenn der Absolutwinkelsensor 20 aus zwei Beschleunigungssensoren für senkrecht aufeinanderstehende Beschleunigungskomponenten besteht und die Beschleunigungskomponenten separat ausgebbar sind, kann der vertikal zurückgelegte Weg und der horizontal zurückgelegte Weg des Hauptfußteils 10 festgestellt werden. Die Wegstrecken werden durch zweifache Integration über die entsprechenden Beschleunigungskomponenten ermittelt. In diesen Fällen ist es möglich, eine Unterscheidung von Treppauf- und Treppabgehen vorzunehmen und dementsprechend die Anschläge für die Dämpfungseigenschaften bei Plantar- und Dorsalflexion einzustellen.

In ähnlicher Weise können die Beschleunigungen ausgenutzt werden, um das Gehen bei unterschiedlichen Ganggeschwindigkeiten, einzustellen, indem die Anschläge nach dorsal sowie die Dämpfungseigenschaften bei Plantar- und Dorsalflexion entsprechend geändert werden.

## Patentansprüche

1. Verfahren zur Steuerung eines orthopädisches Fußteils, das ein Unterschenkel-Anschlussteil, ein als Knöchelgelenk (5) wirkendes Drehgelenk (5), mit dem ein Fußteil (10) in Richtung Dorsalflexion und Plantarflexion drehbar mit dem Anschlussteil verbunden ist, eine die Drehbewegung um das Drehgelenk (5) beeinflussende Dämpfungsanordnung (17), eine Sensoranordnung (7, 20, 21) zur Erkennung von Aktionszuständen des orthopädischen Fußteils und eine mit der Sensoranordnung verbundene Steuerung aufweist, die die Dämpfungsanordnung steuert, **dadurch gekennzeichnet, dass** ein zum am Knöchelgelenk (5) auftretenden Drehmoment proportionaler Wert, der Knöchelwinkel zwischen Anschlussteil und Fußteil (10) und der Absolutwinkel des Fußteils (10) bezogen auf die Lotrechte bestimmt werden, dass in Abhängigkeit von diesen gemessenen Werten das Abrollen des Fußes in der Standphase des Gehens, die Stellung des Fußteils (10) in der Schwungphase des Gehens und die Stellung und Bewegbarkeit des Fußteils (10) während des Stehens ausschließlich mittels der Dämpfungsanordnung (17) gesteuert wird, dass die Beeinflussung von Parametern eines Gangzyklus durch für diesen Gangzyklus bereits gemessene Sensorsignale erfolgt und dass ein Nulldurchgang des Knöchelmoments als Kriterium bei den Messungen verwendet wird.

2. Verfahren nach Anspruch 1, **dadurch gekennzeichnet, dass** zur Steuerung der Dämpfung in Richtung Dorsalflexion und in Richtung Plantarflexion separate Dämpfungsmittel verwendet und gesteuert werden.

3. Verfahren nach Anspruch 2, **dadurch gekennzeichnet, dass** die Dämpfungsmittel während des Gehvorgangs in zahlreiche Zwischenstellungen, vorzugsweise kontinuierlich, verstellt werden.

4. Verfahren nach Anspruch 3, **dadurch gekennzeichnet, dass** die Verstellung der Dämpfungsmittel bis zum Blockieren des Drehgelenks (5) unter Berücksichtigung des gemessenen Knöchelwinkels und/oder Absolutwinkels erfolgt.

5. Verfahren nach einem der Ansprüche 1 bis 4, **dadurch gekennzeichnet, dass** die Beeinflussung von Parametern der Standphase des Gangzyklus durch bereits gemessene Sensorsignale für dieselbe Standphase erfolgt.

6. Verfahren nach einem der Ansprüche 1 bis 5, **dadurch gekennzeichnet, dass** während der Standphase des Gangzyklus maximale Knöchelwinkel für die Dorsalflexion festgelegt werden und dass das betreffende Dämpfungsmittel bei Annäherung an den maximalen Knöchelwinkel seinen Dämpfungswert zunehmend in Richtung Blockieren erhöht.

7. Verfahren nach einem der Ansprüche 1 bis 6, **dadurch gekennzeichnet, dass** während der Standphase des Gangzyklus das Dämpfungsmittel für die Bewegung in Richtung Plantarflexion mit zunehmendem Knöchelwinkel in diese Richtung in seinem Dämpfungswert progressiv erhöht wird.

8. Verfahren nach einem der Ansprüche 1 bis 7, **dadurch gekennzeichnet, dass** ein Zustand "Fersenauftritt" durch Auftreten eines negativen Knöchelmoments in Verbindung mit einer Knöchelwinkelbewegung in Richtung Plantarflexion detektiert wird.

9. Verfahren nach einem der Ansprüche 1 bis 8, **dadurch gekennzeichnet, dass** für das Stehen eine hohe Progression der Dämpfungswerte der Dämpfungsmittel bereits für kleine Winkel nahe dem Nullpunkt eingestellt wird.

10. Verfahren nach Anspruch 9, **dadurch gekennzeichnet, dass** der Nullpunkt in Abhängigkeit von der Neigung einer Auflagefläche des Fußes bzw. der Absatzhöhe eines Schuhs festgestellt wird.

11. Verfahren nach einem der Ansprüche 1 bis 10, **dadurch gekennzeichnet, dass** beim Fersenauftritt das Dämpfungsmittel in Richtung Plantarflexion gesteuert bis zu einem maximalen Dämpfungswert verstellt wird und das beim Übergang zu einem positiven Knöchelmoment das Dämpfungsmittel in Richtung Dorsalflexion zur gesteuerten Rückführung der Plantarflexion und zur Ausbildung eines Anschlags gesteuert wird.

12. Verfahren nach einem der Ansprüche 1 bis 11, **dadurch gekennzeichnet, dass** zu Beginn der Schwungphase des Gangzyklus mittels einer Rückstellfeder eine Rückstellung in Dorsalflexionsrichtung durch Verringerung des Wertes des Dämpfungsmittels in Dorsalrichtung vorgenommen wird, sodass beim Fersenauftritt am Ende der Schwungphase eine Dorsalflexion des Fußteils (10) vorliegt.

13. Verfahren nach einem der Ansprüche 1 bis 12, **dadurch gekennzeichnet, dass** als Fußteil ein an das Drehgelenk angeschlossenes Basisteil und ein damit über ein Drehgelenk verbundenes Vorderfußteil (15) verwendet wird und dass eine Winkelmessung für den Vorfußwinkel des Vorderfußteils (15) relativ zum Basisteil (10) vorgenommen wird.

14. Verfahren nach Anspruch 13, **dadurch gekennzeichnet, dass** zwischen einer Neigung des Untergrunds nach vorn und einem erhöhten Schuhabsatz durch Feststellung des Winkels zwischen Basisteil (10) und Vorderfußteil (15) unterschieden wird.

15. Orthopädisches Fußteil mit einem Unterschenkel-Anschlussteil, einem als Knöchelgelenk (5) wirkenden Drehgelenk (5), mit dem ein Fußteil (10) in Richtung Dorsalflexion und Plantarflexion drehbar mit dem Anschlussteil verbunden ist, mit einer die Drehbewegung um das Drehgelenk (5) beeinflussenden Dämpfungsanordnung (17), mit einer Sensoranordnung (7, 20, 21) zur Erkennung von Aktionszuständen des orthopädischen Fußteil (10) und mit einer mit der Sensoranordnung verbundenen Steuerung, die die Dämpfungsanordnung (17) steuert, **dadurch gekennzeichnet, dass** die Sensoranordnung (7, 20, 21) und die mit der Sensoranordnung verbundene Steuerung zur Durchführung des Verfahrens nach einem der Ansprüche 1 bis 14 eingerichtet sind.

## Claims

1. Method for controlling an orthopaedic foot part which has a connection part for the lower leg, a swivel joint (5) acting as an ankle joint (5) by means of which a foot part (10) is rotatably connected in the direction of dorsiflexion and the direction of plantar flexion to the connection part, a damping arrangement (17) influencing the rotational movement about the swivel joint (5), a sensor arrangement (7, 20, 21) for detecting action states of the orthopaedic foot part, and a control unit connected to the sensor arrangement which controls the damping arrangement, **characterized in that** a value proportional to the torque occurring at the ankle joint (5), the ankle angle between the connection part and the foot part (10), and the absolute angle of the foot part (10) with respect to the plumb line are determined, that in dependence on these measured values the heel-toe motion during the stance phase of walking, the positioning of the foot part (10) in the swing phase of walking and the positioning and the movability of the foot part (10) whilst standing are controlled by means of the damping arrangement alone, that the influencing parameters of a gait cycle is performed with sensor signals already measured for said gait cycle and that a zero crossing of the ankle torque is used as a criterion for measurements.

2. Method according to Claim 1, **characterized in that** separate damping means are used and controlled for controlling the damping in the direction of dorsiflexion and in the direction of plantar flexion.

3. Method according to Claim 2, **characterized in that** the damping means are adjusted, preferably continuously, into a plurality of intermediate positions during the walking process.

4. Method according to Claim 3, **characterized in that** the damping means are adjusted until the swivel joint (5) is blocked, taking into account the measured ankle angle and/or absolute angle.

5. Method according to one of Claims 1 to 4, **characterized in that** the parameters of the stance phase of the gait cycle are influenced by previously measured sensor signals for said stance phase.

6. Method according to one of Claims 1 to 5, **characterized in that** maximum ankle angles for dorsiflexion are determined during the stance phase of the gait cycle and **in that** the relevant damping means raises its damping value up to blocking as the maximum ankle angle is approached.

7. Method according to one of Claims 1 to 6, **characterized in that**, during the stance phase of the gait cycle, the damping value of damping means for movement in the direction of plantar flexion is progressively raised as the ankle angle increases in this direction.

8. Method according to one of Claims 1 to 7, **characterized in that** a "heel impact" state is detected by the occurrence of a negative ankle moment in connection with an ankle-angle movement in the direction of plantar flexion.

9. Method according to one of Claims 1 to 8, **characterized in that**, in the case of standing, a high progression of damping values of the damping means is already set for small angles in the vicinity of the neutral position.

10. Method according to Claim 9, **characterized in that** the neutral position is determined as a function of the incline of a bearing surface of the foot or of the heel height of a shoe.

11. Method according to one of Claims 1 to 10, **characterized in that**, in the case of a heel impact, the damping means is controlled in the direction of plantar flexion and adjusted up to a maximum damping value and that, during the transition to a positive ankle moment, the damping means is controlled in the direction of dorsiflexion for the controlled return of the plantar flexion and for forming a stop.

12. Method according to one of Claims 1 to 11, **characterized in that**, at the outset of the swing phase of the gait cycle, a return spring effects a return in the direction of dorsiflexion by reducing the value of the damping means in the dorsal direction such that there is a dorsiflexion of the foot part (10) during the heel impact at the end of the swing phase.

13. Method according to one of Claims 1 to 12, **characterized in that** a base part, which is connected to the swivel joint, and a forefoot part (15), which is connected to the swivel joint via the former, are used as the foot part, and **in that** an angle measurement is undertaken for the forefoot angle of the forefoot part (15) relative to the base part (10).

14. Method according to Claim 13, **characterized in that** an incline of the ground towards the front and an increased shoe heel are distinguished by determining the angle between the base part (10) and the forefoot part (15).

15. Orthopaedic foot part with a connection part for the lower leg, a swivel joint (5) acting as an ankle joint (5) by means of which a foot part (10) is rotatably connected in the direction of dorsiflexion and the direction of plantar flexion to the connection part, with a damping arrangement (17) influencing the rotational movement about the swivel joint (5), with a sensor arrangement (7, 20, 21) for detecting action states of the orthopaedic foot part (10), and with a control unit connected to the sensor arrangement which controls the damping arrangement (17), **characterized in that** the sensor arrangement (7, 20, 21) and the control unit connected to the sensor arrangement are designed for executing the method according to one of the claims 1 o 14.

## Revendications

1. Procédé pour la commande d'une partie de pied orthopédique, qui comprend une partie de liaison de jambe, une articulation rotative (5) fonctionnant comme une articulation de cheville (5) et au moyen de laquelle une partie de pied (10) est reliée à la partie de liaison avec possibilité de rotation en direction d'une flexion dorsale et en direction d'une flexion plantaire, un agencement amortisseur (17) qui influence le mouvement de rotation autour de l'articulation rotative (5), un agencement capteur (7, 20, 21) pour reconnaître des états d'action de la partie de pied orthopédique, et une commande reliée à l'agencement capteur, qui commande l'agencement amortisseur,
**caractérisé en ce qu'**une valeur proportionnelle au couple de rotation surgissant à l'articulation de cheville (5) détermine l'angle de cheville entre la partie de liaison et la partie de pied (10) et l'angle absolu de la partie de pied (10) par référence à la verticale, et **en ce que** l'on commande, en fonction de ces valeurs mesurées, le roulement du pied dans la phase stationnaire de la marche, la position de la partie de pied (10) dans la phase de propulsion de la marche, et la position et la mobilité de la partie de pied (10) pendant la station debout uniquement par action sur l'agencement amortisseur (17), **en ce que** l'influence sur des paramètres d'un cycle de marche a lieu au moyen de signaux de capteurs déjà mesurés pour ce cycle de marche, et **en ce que** l'on utilise un passage à zéro du couple à la cheville à titre de critère lors des mesures.

2. Procédé selon la revendication 1, **caractérisé en ce que** pour la commande de l'amortissement en direction d'une flexion dorsale et en direction d'une flexion plantaire on utilise des moyens amortisseurs séparés et on les commande.

3. Procédé selon la revendication 2, **caractérisé en ce que** les moyens amortisseurs sont réglés pendant le processus de marche dans un grand nombre de positions intermédiaires, de préférence de manière continue.

4. Procédé selon la revendication 3, **caractérisé en ce que** le réglage des moyens amortisseurs a lieu jusqu'au blocage de l'articulation rotative (5) et en tenant compte de l'angle de cheville mesuré et/ou de l'angle absolu.

5. Procédé selon l'une des revendications 1 à 4, **caractérisé en ce que** l'influence des paramètres de la phase stationnaire du cycle de marche a lieu au moyen de signaux de capteurs déjà mesurés pour la même phase stationnaire.

6. Procédé selon l'une des revendications 1 à 5, **caractérisé en ce que** des angles de cheville maximum pour la flexion dorsale sont fixés pendant la phase stationnaire du cycle de marche, et **en ce que** les moyens amortisseurs concernés augmentent leur valeur d'amortissement progressivement en direction d'un blocage lorsqu'on s'approche de l'angle de cheville maximum.

7. Procédé selon l'une des revendications 1 à 6, **caractérisé en ce que**, pendant la phase stationnaire du cycle de marche, les moyens amortisseurs augmentent progressivement leur valeur d'amortissement pour le mouvement en direction d'une flexion plantaire au fur et à mesure de l'augmentation de l'angle de cheville dans cette direction.

8. Procédé selon l'une des revendications 1 à 7, **caractérisé en ce que** l'on détecte un état "contact de talon" par apparition d'un couple de cheville négatif en association avec un mouvement angulaire de la cheville en direction d'une flexion plantaire.

9. Procédé selon l'une des revendications 1 à 8, **caractérisé en ce que** l'on établit pour la station debout une progression élevée des valeurs d'amortissement des moyens amortisseurs déjà pour des petits angles à proximité du point neutre.

10. Procédé selon la revendication 9, **caractérisé en ce que** le point neutre est déterminé en fonction de l'inclinaison d'une surface d'appui du pied, ou respectivement de la hauteur du talon d'une chaussure.

11. Procédé selon l'une des revendications 1 à 10, **caractérisé en ce que** lors du contact du talon, les moyens amortisseurs sont réglés de façon commandée en direction d'une flexion plantaire jusqu'à une valeur d'amortissement maximum, et **en ce que**, lors d'une transition vers un couple de cheville positif, les moyens amortisseurs sont commandés en direction d'une flexion dorsale pour un retour commandé de la flexion plantaire et en vue de la réalisation d'une butée.

12. Procédé selon l'une des revendications 1 à 11, **caractérisé en ce que**, au commencement de la phase de propulsion du cycle de marche, on procède au moyen d'un ressort de rappel à un rappel en direction de flexion dorsale par réduction de la valeur des moyens amortisseurs en direction dorsale, de sorte que lors du contact du talon à la fin de la phase de propulsion, il existe une flexion dorsale de la partie de pied (10).

13. Procédé selon l'une des revendications 1 à 12, **caractérisé en ce que** l'on utilise à titre de partie de pied une partie de base reliée à l'articulation rotative et une partie d'avant-pied (15) reliée à la partie de base via une articulation rotative, et **en ce que** l'on exécute une mesure angulaire pour l'angle de la partie d'avant-pied (15) par rapport à la partie de base (10).

14. Procédé selon la revendication 13, **caractérisé en ce que** l'on fait une différence entre une inclinaison du sol vers l'avant et un talon de chaussure surélevé par constatation de l'angle entre la partie de base (10) et la partie d'avant-pied (15).

15. Partie de pied orthopédique, comprenant une partie de liaison de jambe, et une articulation tournante (5) fonctionnant comme une articulation de cheville (7) et au moyen de laquelle une partie de pied (10) est reliée à la partie de liaison avec possibilité de rotation en direction d'une flexion dorsale et d'une flexion plantaire, comprenant un agencement amortisseur (17) qui influence le mouvement de rotation autour de l'articulation tournante (5), comprenant un agencement capteur (7, 20, 21) pour reconnaître des états d'action de la partie de pied (10) orthopédique, et comprenant une commande reliée à l'agencement capteur, qui commande l'agencement amortisseur (17), **caractérisée en ce que** l'agencement capteur (7, 20, 21) et la commande reliée à l'agencement capteur sont organisés pour mettre en oeuvre le procédé selon l'une des revendications 1 à 14.
